# EUROPÄISCHE PATENTSCHRIFT

(11) **EP 1 317 254 B1**
(45) Veröffentlichungstag und Bekanntmachung des Hinweises auf die Patenterteilung: **28.02.2007**
(21) Anmeldenummer: 01971697.6
(22) Anmeldetag: 04.09.2001
(51) Int. Cl.: A61K 9/16

(54) **RETARDPARTIKELDISPERSION**
SUSTAINED RELEASE PARTICLE DISPERSION
DISPERSION DE PARTICULES A LIBERATION PROLONGEE

(30) Priorität: 05.09.2000 DE 10044545
(43) Veröffentlichungstag der Anmeldung: 11.06.2003
(73) Patentinhaber: Bodmeier, Roland, 14163 Berlin (DE)
(72) Erfinder: Bodmeier, Roland, 14163 Berlin (DE)
(74) Vertreter: Bohmann, Armin K.
(86) Internationale Anmeldenummer: PCT/DE2001/003438
(87) Internationale Veröffentlichungsnummer: WO 2002/019988

(56) Entgegenhaltungen:
- WO-A-01/35929
- DE-C- 4 122 591
- GB-A- 2 310 801
- US-A- 5 654 008
- SAH H ET AL: "A novel method of preparing PLGA microcapsules utilizing methylethyl ketone." PHARMACEUTICAL RESEARCH , Bd. 13, Nr. 3, 1996, Seiten 360-367, XP008002100 ISSN: 0724-8741
- LAMBERT W J ET AL: "Development of an in situ forming biodegradable poly-lactide-co-glycolide system for the controlled release of proteins." JOURNAL OF CONTROLLED RELEASE, Bd. 33, Nr. 1, 1995, Seiten 189-195, XP004037653 ISSN: 0168-3659

## Beschreibung

Die Erfindung betrifft ein Kit zur Herstellung von Zubereitungen mit retardierter Wirkstofffreisetzung, die mit Vorteil als Arzneimittel, im Pflanzenschutz, in Lebensmitteln und anderen Produkten eingesetzt werden können. Es betrifft insbesondere flüssige Zubereitungen, in denen Retardpartikel dispergiert vorliegen. Es werden derartige Zubereitungen beschrieben, die als Einzel- oder Mehrfachdosen vorliegen und als solche aus flüssigen Vorprodukten hergestellt werden. Ein weiterer Aspekt der Erfindung betrifft ein Verfahren zur Herstellung der Zubereitungen.

Das Interesse an der Entwicklung von Retardzubereitungen, insbesondere von solchen auf der Basis bioabbaubarer Polymere, hat in den letzten Jahren stark zugenommen. Arzneimittel auf der Basis dieser Zubereitungen können therapeutisch effektive Wirkstoffspiegel über Wochen oder Monate für Arzneistoffe mit geringer oraler Bioverfügbarkeit oder hohem First-Pass-Effekt erreichen. Dies trifft insbesondere für die neue Generation der hochpotenten biotechnologisch hergestellten Arzneistoffe wie Peptide oder Proteine zu. Durch die längeren Dosierungsintervale werden tägliche Injektionen vermieden und damit die Patientencompliance verbessert.

Zur Retardierung der Wirkstofffreisetzung wird der Wirkstoff in eine bioabbaubare Polymermatrix eingebettet oder mit einer Polymerhülle umgeben. Bei den wasserunlöslichen Polymeren, die hierfür eingesetzt werden, dominieren aufgrund der bereits erfolgten Zulassung in Arzneimitteln synthetische Poly(laktid-co-glykolid)derivate (PLGA). Die bioabbaubaren Polymere werden in Arzneiformen häufig in Form von Implantaten oder Mikropartikel verarbeitet.

Implantate (Handelspräparate: Profact^{®} Depot, Wirkstoff Buserelinacetat, Firma Hoechst Marion Roussel; Zoladex^{®}, Wirkstoff Goserelinacetat, Firma Zeneca) sind meist durch Schmelzextrusion oder -verpressung hergestellte zylinderförmige Polymerstäbchen mit eingebettetem Wirkstoff. Nachteilig ist bei diesen Verfahren, dass hohe Prozeßtemperaturen zum Arzneistoffabbau führen können, und bei niedrig dosierten Wirkstoffen kann es zu Homogenitätsproblemen kommen. Ferner sind die zur Implantation notwendigen chirurgischen Eingriffe oder die Injektion durch große Kanülen bei Patienten nicht beliebt.

Bioabbaubare Mikropartikel (Handelspräparate: Enantone^{®} Depot, Wirkstoff Leuprorelinacetat, Firma Takeda Chemicals; Decapeptyl^{®} Depot, Wirkstoff Triptorelinacetat, Firma Ferring) können in Form von wässrigen Dispersionen der Mikropartikel leicht injiziert werden und werden daher von Patienten im Vergleich zu Implantaten bevorzugt. Diese Mikropartikel werden durch verschiedene Verfahren wie z. B. Lösungsmittelverdampfungs- oder -extraktionsverfahren ("Solvent evaporation", siehe z. B. EP 0 190 833, oder "Solvent extraction"), organische Phasenseparations- (EP 0 052 510) oder Sprühtrocknungsverfahren (EP 0 505 966) hergestellt.

In der zur Herstellung bioabbaubarer Mikropartikel häufig verwendeten Solvent-Evaporation-Methode wird z. B. ein Arzneistoff in einer Lösung eines bioabbaubaren Polymeren gelöst oder dispergiert (z. B. in PLGA und Dichlormethan). Diese arzneistoffhaltige Polymerphase wird dann in eine äußere, wässrige Phase zur Bildung wirkstoffhaltiger Polymertröpfchen emulgiert. Die Mikropartikel werden nach dem Verdampfen des Lösungsmittels oder nach dessen Diffusion in die äußere Phase durch Verfestigung des Polymers erhalten und dann von der wässrigen Phase z. B. durch Filtration abgetrennt und getrocknet.

Bei der organischen Phasenseparation wird die Koazervation zu Partikeln durch Zugabe eines Nichtlösungsmittels (z. B. Silikonöl) zu einer Dispersion des Wirkstoffes in einer organischen Polymerlösung induziert. Die Mikropartikel werden gehärtet, filtriert, gewaschen und getrocknet.

Bei der Sprühtrocknung werden die Mikropartikel durch das Versprühen/Trocknen einer wirkstoffhaltigen organischen Polymerlösung in einem erwärmten Luftstrom und Abtrennung in einem Zyklon erhalten.

Handelspräparate bioabbaubarer Mikropartikel bestehen meist aus einem Trockenpulver der Mikropartikel und einem davon getrennt aufbewahrten wässrigen Suspensionsvehikel. Aufgrund der hydrolytischen Instabilität der bioabbaubaren Polymere werden die Mikropartikel und das wässrige Suspensionsvehikel getrennt, z. B. in Zweikammerspritzen oder in zwei Ampullen, aufbewahrt. Die Mikropartikel werden dann kurz vor der Anwendung in dem wässrigen Suspensionsvehikel suspendiert und dann meist subkutan oder intramuskulär injiziert.

Bei den aufgeführten Mikroverkapselungsverfahren und den damit hergestellten Mikropartikeln können verschiedene Probleme auftreten: (1) der Einsatz toxischer organischer Lösungsmittel bzw. im Produkt verbleibende Lösungsmittelrückstände; (2) eine Vielzahl zu validierender Prozeß- und Formulierungsparameter, welche die Eigenschaften des Mikropartikelpräparates beeinflussen; (3) eine niedrige Verkapselungseffizienz oder Produktausbeute; (4) die Notwendigkeit aufwendiger Maßnahmen zur aseptischen Herstellung oder die Gefahr von Veränderungen der Partikeleigenschaften nach einer Strahlensterilisation des Endprodukts; (5) Stabilitätsprobleme während der Lagerung der Partikel, z. B. Veränderung der Freisetzungsprofile aufgrund von Alterungsprozessen. In manchen Fällen werden die Mikropartikel aufgrund ihrer Hydrolyselabilität getrennt vom wässrigen Suspensionsvehikel in Ampullen oder Zweikammerspritzen gelagert. Eine derartige Lagerung führt zur Notwendigkeit eines Rekonstitutionsschrittes kurz vor der Applikation. Die Suspendierung der Mikropartikel mit nachfolgender Injektion kann aber ebenfalls mit Schwierigkeiten verbunden sein, z. B. mit einer Agglomeration der Partikel, dem Verbleiben von Mikropartikelrückstände in der Spritze, dem Verstopfen der Kanüle etc.

In der WO 98/55100 werden Zubereitungen beschrieben, die einige der Probleme vermeiden, die bei der Herstellung und Anwendung von Mikropartikeln und Implantaten auftreten. Dabei handelt es sich um Zubereitungen, die beim Einbringen in den Körper eines Menschen oder eines Tieres Partikel und/oder Implantate bilden. Die Zubereitungen enthalten eine innere, polymerhaltige Trägerphase und eine mit dieser nicht oder teilweise nicht mischbaren äußeren Phase, wobei sich durch die z. B. nach der Injektion der Zubereitung erfolgenden Veränderungen der Umgebungsbedingungen die Viskosität der Trägerphase so verändert, dass eine Verfestigung des Polymers zu Partikeln oder Implantaten eintritt.

Nach der WO 98/55100 kann z. B. eine wirkstoffhaltige Dispersion aus einer inneren, arzneistoffhaltigen Trägerphase und einer zweiten, äußeren Phase (z. B. Öl) hergestellt und in den Körper eingebracht werden. Die innere Trägerphase enthält eine Lösung aus einem Polymer (z. B. PLGA) und einem Lösungsmittel. Nach Einbringen in den Körper kommt es z.B. zu einer Verfestigung der inneren Phase, z. B. durch Lösungsmitteldiffusion in die Umgebung oder Eindiffusion von Körperflüssigkeiten. Die Dispersion kann z. B. im Falle von bioabbaubaren Polymeren i.m. oder s.c. injiziert werden. Im Kontakt mit Körperflüssigkeiten verfestigt sich die innere Phase und bildet u.a. Partikel.

Es besteht ein Bedarf für Retardzubereitungen, welche die Nachteile des Standes der Technik, z.B. die sehr aufwendige großtechnische Herstellung von Mikropartikeln nicht aufweisen. Insbesondere bleibt ein Bedarf für Zubereitungen mit Retardpartikeln, die erst kurz vor der Applikation aus Vorprodukten hergestellt werden können.

Aufgabe der Erfindung ist die Bereitstellung einer flüssigen Zubereitung mit Retardpartikeln, welche die Nachteile des Standes der Technik nicht besitzt und die sich auf einfache Art dosisweise applizieren läßt.

Die Lösung der Aufgabe gelingt durch ein Kit zur Herstellung einer gebrauchsfertigen Einzel- oder Mehrfachdosis einer pharmazeutischen Dispersion von Retardpartikeln, enthaltend mindestens zwei zur Herstellung einer Einzel- oder Mehrfachdosis portionierte Vorprodukte, dadurch gekennzeichnet, dass mindestens zwei der Vorprodukte flüssig sind und die Retardpartikel durch Mischen der flüssigen Vorprodukten gebildet werden.

Unter einer gebrauchsfertigen Zubereitung ist eine unmittelbar verwendungsfähige bzw. applizierbare Komposition aus mindestens einem Wirkstoff und Hilfsstoffen zu verstehen. Im Sinne der Erfindung liegt die Zubereitung dosisexakt bemessen oder portioniert vor, d. h. entweder als Einzeldosis oder als eine definierte Mehrfachdosis. Eine Mehrfachdosis besteht aus dem exakt bemessenen Mehrfachen (z. B. einem 2fachen, 10fachen oder 50fachen) einer Einzeldosis; hierin und ggf. in der Lagerfähigkeit unterscheidet sie sich von Bulkware.

Die Zubereitung weist eine retardierte Wirkstofffreisetzung auf. Unter "retardiert" sollen hier alle Arten von Freisetzungsprofilen außer einer schnellen, ungebremsten Freisetzung verstanden werden, z. B. eine kontrollierte Freisetzung, eine mehrphasige Freisetzung, eine verzögerte Freisetzung usw. Eine retardierte Freisetzung ist gegeben, wenn nur ein Wirkstoff oder wenn alle enthaltenen Wirkstoffe retardiert freigesetzt werden. Die Freisetzung kann über mehrere Stunden, Wochen, Monate oder sogar Jahre geschehen.

Eine Dispersion von Retardpartikeln im Sinne der Erfindung ist ein mehrphasiges System mit flüssiger (auch dickflüssiger) äußerer Phase und darin verteilten festen oder halbfesten Partikeln, die Wirkstoff enthalten und retardiert freisetzen, als innere Phase. Der Begriff Partikel ist weitgreifend, z. B. in Bezug auf Form, Größe, Konsistenz und Aufbau. Die Partikelgröße hängt unter anderem von den Scherkräften während des Mischvorganges, des Mischkörpers, den Viskositäten der Trägerphase (abhängig z. B. von der Konzentration und Molmasse des Trägermaterials) und der zweiten Phase (z. B. Zusatz von viskositätserhöhenden Substanzen zu einer wässrigen Phase) und dem Verfestigungsprozeß der Partikel ab. Die Partikelgröße kann dabei eine Bereich von über einem 1 mm bis in den kolloidalen Bereich (< 1µm, Nanopartikel, Nanokapseln) abdecken. Für parenterale Depotarzneiformen sind meist Teilchengrößen unter 100 µm bevorzugt. Die Partikel entstehen durch Verfestigung der Trägerphase während des Mischvorganges. Innerhalb der Partikel können auch Domainen mit unterschiedlichem Verfestigungsgrad (Konsistenz) vorliegen, z. B. kann die Partikeloberfläche einen anderen Verfestigungsgrad als das Partikelinnere haben. Der Aufbau der Partikel kann auch vielfältig sein und kann z. B. Reservoir- (Kapseln) oder Matrixstrukturen aufweisen oder auch eine unterschiedliche Poren- /Kanalstruktur innerhalb der Partikel aufweisen, oder aus Agglomeraten von Partikeln bestehen. Eine Vielfalt von unterschiedlichen Partikelstrukturen ist möglich.

Die Zubereitung weist flüssige Vorprodukte aus. Unter "flüssig" soll hier ein weiter Viskositätsbereich der Vorprodukte, z.B. neben dünnflüssigen auch halbfeste, gelartige, oder pastöse Vorprodukte, verstanden sein.

Die Retardpartikel entstehen durch Mischen mindestens zweier flüssiger, zur Herstellung der Einzel- oder Mehrfachdosis portioniert vorliegender Vorprodukte. Demnach können mehr als zwei Vorprodukte zur Herstellung der Retardpartikel verwendet werden; mindestens zwei von ihnen sind jedoch flüssig. Eines oder mehrere weitere Vorprodukte können flüssig oder fest sein. Erfindungsgemäß liegen die Vorprodukte bereits portioniert zur Herstellung einer Einzel- bzw. Mehrfachdosis vor, als ein Kit. Bevorzugt liegen sie für eine Einzeldosis portioniert vor.

Im Gegensatz zu den aufwendigen Herstellungsverfahren für Retardpartikel nach dem Stand der Technik können die Mikropartikel also kurz vor der Anwendung z. B. durch medizinisches Fachpersonal aus den Vorprodukten hergestellt und im Anschluss daran appliziert werden, z. B. durch i.m.- oder s.c.-Injektion.

Gemäß der Erfindung kann eine Zubereitung z. B. aus einer arzneistoffhaltigen Poly(lactid-co-glycolid)-Lösung in einem biokompatiblen Lösungsmittel wie Ethylacetat als erstem flüssigen Vorprodukt in einer Spritze und einem davon getrennt aufbewahrten wässrigen Dispersionsvehikel (z. B. Wasser für Injektionszwecke, Tween 80, Natrium Carboxymethylcellulose) als zweitem flüssigen Vorprodukt in einer zweiten Spritze (zweite Phase) hergestellt werden. Die Partikeldispersion wird vor der Anwendung über ein Verbindungsstück, das die beiden Spritzen verbindet (oder anderweitig zusammengesteckte, -geschraubte Spritzen), durch das Mischen der beiden flüssigen Vorprodukte durch Hin- und Herschieben der Spritzenkolben gebildet. Dabei bildet sich eine Dispersion aus der polymerhaltigen Trägerphase (innere Phase) und einer wässrigen zweiten Phase (externe Phase). Die arzneistoffhaltigen Polymerpartikel (innere Phase) bilden sich dabei z. B. durch Verfestigung der Polymerlösung, bedingt durch eine Wegdiffusion des Lösungsmittels in die externe, wässrige Phase und eine Eindiffusion der wässrigen Phase in die Polymertröpfchen. Diese Partikeldispersion, die im Falle einer Verwendung eines Lösungsmittels in der Trägerphase auch dieses Lösungsmittel enthält, wird dann s.c. oder i.m. in den Patienten injiziert und setzt dort den Arzneistoff retardiert frei.

Vorzugsweise erfolgt die Herstellung der Zubereitung und die Bildung der Retardpartikel erst kurz vor der Applikation, d. h. in einem Zeitintervall vor der Applikation, in dem üblicherweise auch die Rekonstitution bereits vorgefertigter Retardpartikel mit einem Dispersionsvehikel erfolgt. Besonders bevorzugt ist eine Herstellung am Tage der Applikation, insbesondere jedoch innerhalb von etwa 2 bis 120 Minuten vor der Applikation.

Die Partikelbildung kann z. B. durch eine Konzentrierung eines polymeren Trägermaterials, eine Ausfällung des Trägermaterials, eine Wegdiffusion des Lösungsmittels für das Polymer in die aus dem zweiten flüssigen Vorprodukt gebildete Phase, eine Temperaturänderung, eine pH-Änderung, eine Veränderung der Ionenstärke oder - art, oder durch eine Kombination von zwei oder mehreren der genannten Vorgänge erfolgen.

Vorzugsweise wird die aus dem Mischen der Vorprodukte resultierende Zubereitung mit den Retardpartikeln ohne weitere Aufbereitungsschritte, insbesondere ohne Anreicherung der Partikel oder deren partielle oder vollständige Abtrennung von flüssiger Phase durch Trocknen, Zentrifugieren, Dialysieren, Filtrieren usw. appliziert.

In einer bevorzugten Ausführung der Erfindung ist vorgesehen, dass die flüssigen Vorprodukte getrennt in separaten Kammern lagerfähig primärverpackt vorliegen. Dabei können die separaten Kammern Teile derselben oder verschiedener Vorrichtung sein, also etwa in verschiedenen Spritzen oder in einer Mehrkammerspritze. Die flüssigen Vorprodukte können vor der Applikation durch Zusammenstecken/-schrauben der Spritzen (z. B. über ein Verbindungsstück) oder innerhalb der Mehrkammerspritze gemischt werden. Die Vorprodukte können natürlich auch in sonst üblichen Behältnissen (z. B. Ampullen oder Glasvials) getrennt aufbewahrt werden und dann kurz vor der Anwendung gemischt werden. Wenn es sich um eine injizierbare Zubereitung handelt, sollen die Vorprodukte steril verpackt vorliegen und die Kammern vorzugsweise dazu geeignet sein, den Mischvorgang unter aseptischen Bedingungen zu ermöglichen.

In der Regel enthält eines der mindestens zwei flüssigen Vorprodukte ein polymeres Trägermaterial, das in dem betreffenden Vorprodukt (Trägerphase) gelöst und/oder dispergiert vorliegt oder die Trägerphase selbst bildet. Durch das Mischen der Vorprodukte wird eine Verfestigung des Trägermaterials ausgelöst, wodurch sich die Retardpartikel bilden. Somit bilden das Trägermaterial einen wichtigen Bestandteil der Partikel.

Das Trägermaterial ist vor allem für die Retardierung der Wirkstofffreisetzung wichtig. Als Trägermaterial eignen sich je nach spezifischer Anforderung z. B. an die Freisetzungszeit wasserlösliche oder wasserunlösliche Polymere, in wässrigen Flüssigkeiten lösliche Polymere, biokompatible und/oder bioabbaubare Polymere, Proteine, Lipide, Wachse, nichtpolymere Materialien oder Kombinationen der genannten Materialien.

Die Trägermaterialen sind synthetischen, halbsynthetischen und natürlichen Ursprungs. Zu den bevorzugten Trägermaterialien zählen Cellulosederivate (z. B. Celluloseacetat, Ethylcellulose, Celluloseacetatphthalat, Celluloseether wie z. B. Hydroxypropylmethylcellulose), Acrylatderivate (z. B. Eudragite, Poly(methylmethacrylat), Cyanoacrylate), biokompatible und bioabbaubare Polymere wie Polyanhydride, Polyester (z. B. Polylaktid, Polyglykolid, Polycaprolacton, Polyhydroxybutyrat-oder valerat oder Derivate dieser Polymere, z. B. Poly(laktid-coglykolid/Polyethylenglykol Blockpolymere), Polyamine, Polyaminosäuren, Polyorthoester, Polyurethane, Poly(ortho)carbonate, Polyphosphazene, Polyacetale, Polyketale, Polyalkylenoxalate, Polyalkylensuccinate, Polyoxyethylen-oxypropylen, Polysaccharide (z. B. Na Alginate, Chitosan oder Chitin), Polyethylenglycol, Polyvinylpyrrolidon, Lipide (z.B. Wachse, Fette, Glyceride) und Copolymere oder Terpolymere oder Mischungen aus zwei oder mehreren der genannten Trägermaterialien. Ein Vielzahl von Trägermaterialien sind in der Literatur beschrieben und dem Fachmann als einsatzfähige Bestandteile von Retardpartikeln bekannt. Besonders geeignete Trägermaterialien sind Polylaktid, Polylaktid-Derivate, Polyanhydride und Polyorthoester.

In der Regel, nämlich dann, wenn das Trägermaterial selbst nicht flüssig oder verflüssigbar vorliegt, enthält das trägermaterialhaltige Vorprodukt (Trägerphase) Wasser und/oder ein organisches Lösungsmittel wie Ethanol, Aceton, Methylethylketon, Butanol, Ethylformat, Essigsäure, Milchsäure, Pentanol, n- oder i-Propanol, Tetrahydrofuran, Triethylcitrat, Citratester, Phthalatester, Isopropylmyristat, Triacetin, Tributyrin, Propylenglykol, Glycerol, Polyethylenglykol, Aceton, 2-Ethoxyethylacetat, Ethylacetat, Methylacetat, Ethyllactat, Ethylbutyrat, Benzylalkohol, Benzylbenzoat, Dimethylformamid, Dimethylsulfoxid, Dimethylacetamid, Propylencarbonat, Glykofurol, Solketal, Ölsäure, 2-Pyrrolidon, N-Methyl-2-Pyrrolidon, Caprolactam, ein Öl, einen Weichmacher oder eine Mischung aus zwei oder mehreren der genannten Lösungsmittel als Vehikel für das Trägermaterial.

Es bieten sich vor allem Lösungsmittel aus der "Draft guideline of the international conference on harmonization on impurities - residual solvents" an. Das biokomatible Lösungsmittel sollte das Trägermaterial vorzugsweise (ggf. kolloidal) lösen. Es können auch Kombinationen aus Lösungsmitteln und Nichtlösungsmitteln für das Trägermaterial verwendet werden. Es können auch Lösungsmittelgemische verwendet werden, ausgewählt z. B. nach Lösungsmittelqualität für das Trägermaterial oder Mischbarkeit mit wässrigen Phasen. Durch die Lösungsmittelauswahl kann z. B. die Verfestigung des Trägermaterials und die Mischbarkeit mit Körperflüssigkeiten oder mit dem zweiten flüssigen Vorprodukt beeinflusst werden.

Im Falle von Polymerlösungen (Trägerphase) sollte das Lösungsmittel/Lösungsmittelgemisch vorzugsweise nur begrenzt und nicht vollständig mit dem zweiten flüssigen Vorprodukt mischbar sein (z. B. Löslichkeit von Ethylacetat in Wasser, ca. 10%, Triacetin in Wasser, ca. 8%). Partikel müssen während des Mischvorgangs gebildet werden, und dies legt im Einzelfall den Grenzfall der Mischbarkeit des Lösungsmittels mit der zweiten Phase nach oben und unten fest. Bei einer begrenzten Löslichkeit kommt es durch die Lösungsmitteldiffusion aus der Trägerphase in die durch das zweite flüssige Vorprodukt gebildete Phase zur Verfestigung der Trägerphase bzw. des Trägermaterials. Der Verfestigungsgrad der Trägerphase bzw. des Trägermaterials hängt u. a. auch vom Mengenverhältnis der beiden Phasen ab. Bei einer Dispersionsbildung wird eine Verfestigung der Trägerphase bevorzugt bei einem niedrigerem Verhältnis von Trägerphase zur zweiten Phase eintreten, bei höherem Verhältnis würde die innere Phase dagegen flüssiger bleiben. Eine vollständige Partikelbildung lässt sich z.B. leicht durch mildes Zentrifugieren der Dispersion nachweisen, dabei bilden sich aufschüttelbare Partikel, bei anderen Formulierungen koalesziert die dispergierte Trägerphase.

Liegt das Trägermaterial in der Trägerphase in einem Lösungsmittel gelöst vor, so kann die Viskosität der Trägerphase durch das Trägermaterial selbst (z. B. seine Molmasse, Konzentration etc.) und auch durch das Lösungsmittel beeinflusst werden. Im Falle von Poly(lactid-co-glycolid)-lösungen kann die verarbeitbare Polymerkonzentration der Trägerphase u. a. durch die Molmasse des Polymers beeinflusst werden. Niedermolekulare Polymere ergeben höherkonzentrierte Polymerlösungen (Trägerphase). Höherkonzentrierte Lösungen niedermolekularer Polymere führen unter bestimmten Bedingungen zu einem besseren Wirkstoffeinschluss in die Partikel und zu einer besseren Retardierung der Wirkstofffreisetzung. Mit Poly(lactid-co-glycolid)-Polymeren können mit niedermolekularen Polymeren hochkonzentrierte Lösungen (>50 % [m/m]) verwendet werden.

Das zweite flüssige Vorprodukt kann als wesentliche Bestandteile natürliche, halbsynthetische oder synthetische Fette, Öle oder Wachse wie Baumwollsaatöl, Sojaöl, Saffloröl, hydriertes Erdnußöl, Olivenöl, Rizinusöl, Triglyceridgemische (wie Miglyol), Monoglyceride (wie Glycerolmonooleat), Silikonöl, Isopropylmyristat, Ethyloleat, Paraffine, Wasser, Glycerol, Propylenglycol oder Polyethylenglycol oder Mischungen aus zwei oder mehreren der oben genannten Stoffe enthalten. Ein besonders bevorzugter Bestandteil ist Wasser. In der Regel sind Bestandteile der Trägerphase mit dem zweiten flüssigen Vorprodukt begrenzt mischbar. Bestandteile der Trägerphase und das zweite flüssige Vorprodukt sind mit wässrigen Flüssigkeiten vollständig, begrenzt oder nicht mischbar. Beim Mischen der Vorprodukte bildet das zweite flüssige Vorprodukt meist den Hauptbestandteil der äusseren Phase der sich bildenden Partikeldispersion.

Erfindungsgemäß kann der Wirkstoff z. B. ein Arzneistoff, ein Peptid- oder Proteinarzneistoff, ein Oligonucleotid, oder ein Gentherapeutikum sein und z. B. aus der Gruppe der Antibiotika, Antiinflammatoria, Antiinfektiva, Hormone, immunologisch wirksamen Stoffe, Impfstoffe, Immunmodulatoren, Immunsuppresiva, Zytostatika, Diuretika, Magen-Darm-Mittel, Herz-Kreislauf-Mittel, Respirationstrakt-Mittel, Wachstumsfaktoren, Analgetika, Lokalanaesthetika und/oder Neuropharmaka stammen. Mögliche Arzneistoffe sind z. B. in WO 00/24374 oder WO 98/27963 aufgeführt. Darüber hinaus können mit der erfindungsgemäßen Zubereitung bei Bedarf zwei oder mehr Wirkstoffe verabreicht werden, zumindest einer davon retardiert. Zu den Wirkstoffen zählen neben Arzneistoffen zur human- und veterinärmedizinischen Anwendung auch Substanzen, die in der Landwirtschaft, im Haushalt, in der Nahrungsmittel-, kosmetischen und chemischen Industrie und anderen Industriezweigen genutzt werden.

Erfindungsgemäß liegt der Wirkstoff vorzugsweise in dem ersten flüssigen Vorprodukt (der Trägerphase) gelöst, dispergiert, suspendiert oder emulgiert vor; er kann aber auch zusätzlich oder ausschließlich in der zweiten Phase vorliegen. Durch die Lösungsmittel/Lösungsmittelgemischauswahl kann der physikalische Zustand des Arzneistoffs in der Trägerphase bestimmt werden. Bei Anwesenheit von mehreren Wirkstoffen kann es vorteilhaft sein, dass einer der Wirkstoffe (zumindest überwiegend) im ersten Vorprodukt, ein weiterer (zumindest überwiegend) im zweiten Vorprodukt vorliegt. Bei Stabilitätsproblemen kann ein Wirkstoff auch als Bestandteil eines weiteren flüssigen oder festen Vorprodukts separat gelagert und zur Herstellung der Zubereitung unter Bildung der Retardpartikel einem der beiden erstgenannten flüssigen Vorprodukte oder beiden zugegeben werden. Ein mögliche Variante ist z. B. die getrennte Aufbewahrung von Wirkstoff in fester Form, flüssiger Trägerphase und zweiter flüssiger Phase innerhalb von mehreren Spritzen. Erfindungsgemäß kann der Wirkstoff auch in wässriger Lösung in eines der flüssigen Vorprodukte gegeben werden, bevor dieses mit dem zweiten flüssigen Vorprodukt gemischt wird. Diese Zugabe der wirkstoffhaltigen, wässrigen Phase kann auch unmittelbar vor der Herstellung der Partikel erfolgen, es ist dann die wässrige Phase mit der Trägerphase und dann mit der zweiten Phase zur Partikelbildung zu mischen. Dabei kann die Trägerphase eine w/o-Emulsion bilden, oder der Wirkstoff kann bei Zugabe der wässrigen Lösung zur Trägerphase auch fein dispergiert ausfallen. Dieses Verfahren ermöglicht z. B. die Verkapselung wasserlöslicher Wirkstoffe. Anstatt Wasser sind auch andere Lösungsmittel möglich.

Ferner können in den Vorprodukten auch weitere Hilfsstoffe wie z. B. viskositätserhöhende Substanzen, Substanzen zur Erzeugung eines thixotropen Effekts, Stabilisatoren, Emulgatoren, Freigaberegulatoren, Substanzen, welche die Verweildauer am Applikationsort verändern, bioadhäsive Materialien, Penetrationsverbesserer, Substanzen, die die Löslichkeit des Wirkstoffes beeinflussen, oder Substanzen, die die Partikelbildung (z.B. Partikelstruktur) beeinflussen, enthalten sein, um die Eigenschaften der Zubereitung nach gängigen Methoden der pharmazeutischen Technologie zu beeinflussen, z. B. mit dem Ziel, den Wirkstoff am Applikationsort verzögert freizugeben und/oder eine anfängliche, rasche Freigabe des Wirkstoffes am Applikationsort zu vermeiden. Insbesondere können Stabilisatoren, wie z. B. Emulgatoren notwendig sein, die der Trägerphase und/oder der zweiten Phase beigegeben werden. Zu den Emulgatoren zählen unter anderem Polyethylenglykol-fettsäureester, -fettsäureether, - sorbitanfettsäureester, Sorbitanfettsäureester, Partialfettsäureester mehrwertiger Alkohole oder Zucker, Lecithine, Phospholipide, Poloxamere, Proteine und Cellulosederivate, z. B. Celluloseether.

Der Wirkstoff kann ferner verkapselt in der Trägerphase vorliegen, z. B. in Form von Mikropartikeln, Mikrokapseln, kolloidalen Teilchen (z. B. Nanopartikel, Nanokapseln, Liposomen), er kann auch an Ionenaustauscherharze, Cyclodextrine oder andere Komplexbildner gebunden vorliegen. Im Falle wasserlöslicher Wirkstoffe (z. B. bei Peptid-/Protein-/Oligonucleotidarzneistoffen) ist durch Komplexierung mit entgegengesetzt geladenen nieder- oder hochmolekularen Molekülen (z. B. kationische/anionische/amphotere Lipide, Polysaccharide oder Proteine) eine Verringerung der Löslichkeit der Wirkstoffe in wässrigen Flüssigkeiten möglich. Dadurch kann z. B. ein besserer Einschluss des Wirkstoffes in die Retardpartikel (z. B. im Falle der Verwendung einer wässrigen Phase als zweites Vorprodukt) oder auch eine zusätzliche Retardierung der Freisetzung erreicht werden. Diese schwer wasserlöslichen Arzneistoffkomplexe oder - salze können als Ausgangssubstanz eingesetzt werden. Diese Komplexe können aber auch erst während der Partikelbildung gebildet werden. Dabei kann der schwer lösliche Komplex in die Partikel eingeschlossen, an diese gebunden oder auch adsorbiert sein.

Der Einschluss des Wirkstoffes in die Partikel hängt von vielen Faktoren, z. B. von seiner Löslichkeit/Benetzbarkeit in der Trägerphase oder dem zweiten flüssigen Vorprodukt ab. Zur Erzielung einer ausreichenden Beladung der Partikel stehen dem Fachmann eine Vielzahl galenischer Maßnahmen zur Verfügung, die aus der Literatur über disperse Systeme, wie z. B. Mikropartikel, bekannt sind. Die Löslichkeit des Wirkstoffes in der zweiten Phase kann z. B. durch Zusatz von osmotisch aktiven oder pH-beinflussenden Substanzen verringert werden.

Die erfindungsgemäß hergestellte Zubereitung bewirkt, dass der Wirkstoff am Applikationsort verzögert freigegeben wird. Die Wirkstofffreisetzung kann unter anderem durch die Partikeleigenschaften (u.a. Größe, Aufbau, Verfestigungsgrad), die Wirkstoffbeladung, die Wirkstofflöslichkeit, die Wirkstoffmodifikation (z. B. Polymorphie, Komplexbildung), das Trägermaterial und die Trägermaterialkonzentration beeinflusst werden. Ferner können auch Freigaberegulatoren, wie z. B. hydrophile oder lipophile Stoffe anorganischer, organischer oder polymerer Natur mit eingearbeitet werden. Auch der Aufbau (z. B. Porosität) der Partikel beeinflusst die Freisetzung und kann durch die Auswahl der Trägerphase und der zweiten Phase und entsprechender Zusätze während der Partikelbildung beeinflusst (siehe Literatur zur Herstellung von Mikropartikel mit der Solvent Evaporationsmethode). Der häufig unerwünschte Burst-Effekt (initiale hohe Wirkstofffreisetzung) kann durch dem Fachmann bekannte Hilfsstoffe oder Verfahrensschritte beeinflusst werden.

Die erfindungsgemäß hergestellter Zubereitungen sind vorzugsweise (z. B. durch die Auswahl der Hilfsstoffe und die Vorbehandlung der Vorprodukte) zur parenteralen (z. B. i.v., i.m., s.c., i.a.), peroralen, rektalen, buccalen, ophthalmischen, pulmonalen, vaginalen, nasalen, lokalen, sublingualen, peridontalen oder transdermalen Anwendung und/oder zur Einbringung in Körperöffnungen und/oder zum Aufbringen auf Körperflächen geeignet. Die Zubereitungen können aber auch zur Behandlung von Weich- und Hartgewebedefekten, z. B. als Gerüst, zur Gewebeerneuerung (tissue regeneration), als Klebstoff, oder zur Füllung von Körperkavitäten, oder Behandlung von Wunden eingesetzt werden. Im Falle der inneren oder externen Wundbehandlung kann die Zubereitung vor der Anwendung auf verschiedene medizinisch verwendete Stoffgewebe aufgebracht werden.

Bevorzugt werden die Partikeldispersionen unverändert und ohne weitere Behandlungsschritte unmittelbar nach der Herstellung appliziert. Es werden also fertig applizierbare Partikeldispersionen hergestellt und nicht größere Chargen an Partikeldispersionen, die dann noch in Einzelbehälter vor der Injektion abgefüllt werden.

Die Zubereitung kann durch dem Fachmann bekannte Verfahren in Körperöffnungen oder auf Körperflächen aufgebracht werden (z. B. durch Injektion oder Auf-/Einsprühen). Nachdem Ein-/Aufbringen der Partikeldispersionen kann es zu einer weiteren Veränderung der Konsistenz der Partikel (z. B. eine weitere Verfestigung, die auch in unterschiedlichen Domainen innerhalb der Partikel auftreten kann) nach oben beschriebenen Mechanismen kommen. Auch eine Erweichung der Partikel und ein Zusammenfließen der Partikel ist unter bestimmten Vorraussetzung möglich.

In einem weiteren Aspekt der Erfindung wird ein Verfahren zur Herstellung der oben beschriebenen Zubereitungen offenbart. Das Verfahren besteht darin, dass in einem oder mehreren Schritten die Vorprodukte des oben beschriebenen Kits zur Bildung von Retardpartikeln gemischt werden.

Nach der Herstellung der Partikeldispersion wird diese bevorzugt mit unveränderter Zusammensetzung der Wirkstoffe/Hilfsstoffe appliziert. Denkbar ist aber auch eine Konzentrierung der Partikel in der zweiten, meist externen Phase der Dispersion durch Abtrennung eines Teiles der zweiten Phase oder ein teilweiser bis nahezu vollständiger Ersatz der zweiten Phase durch eine andere externe Phase, die z. B. lösungsmittelfrei und wirkstofffrei ist. Viele Varianten zur Herstellung der Partikeldispersion sind möglich.

Erfindungsgemäß sind ferner Verfahren zur Injektion einer Partikeldispersion, dadurch gekennzeichnet dass a) eine trägermaterialhaltigen Trägerphase mit einer zweiten Phase gemischt wird, b) es bei diesem Mischvorgang zu einer Verfestigung der Trägerphase und zur Bildung der Partikel kommt und c) diese Partikeldispersion kurz nach dem Mischvorgang injiziert wird.

Kits sind Zusammenstellungen von aufeinander abgestimmten und ggf. gemeinsam in einer Sekundärverpackung gelagerten Vorprodukten zur Herstellung oben beschriebener Zubereitungen. Demnach enthält ein erfindungsgemäßes Kit mindestens zwei zur Herstellung einer Einzel- oder Mehrfachdosis portionierte Vorprodukte, wobei mindestens zwei der Vorprodukte flüssig sind und die Retardpartikel durch Mischen der flüssigen Vorprodukte gebildet werden.

Ein Kernpunkt der Erfindung ist also, dass die vom Hersteller (z. B. Arzneimittelhersteller) abgegebene Zubereitung nicht bereits Mikropartikel enthält, sondern dass diese Partikel ggf. erst kurz vor der Applikation (z. B. Injektion) vom Anwender (z. B. medizinisches Fachpersonal) durch Mischen zweier flüssiger Vorprodukte (z. B. einer Trägerphase und einer mit dieser begrenzt mischbaren, zweiten Phase) in Form einer injektions-(applikations-)bereiten Partikeldispersion hergestellt werden. Diese Partikeldispersion wird unmittelbar (bevorzugt innerhalb weniger Minuten, oder innerhalb weniger Stunden) nach der Herstellung appliziert. Im Vergleich zu den gegenwärtig verfügbaren Mikropartikelpräparaten entfällt dabei die komplizierte großtechnische Herstellung der Mikropartikel mit den oben aufgeführten Problemen.

Durch die nachfolgenden Beispiele wird die Erfindung erläutert.

### Beispiel 1

Eine Lösung von Poly(D,L-lactid-co-glycolid) (75/25) (40%m/m) in Ethylacetat (Spritze 1) (200µl) wird mit einer wässrigen Phase (Wasser für Injektionszwecke, Tween 80 (oder alternativ Pluronic F 68), Na Carboxymethylcellulose) (Spritze 2) (2ml) über ein Verbindungsstück oder durch Zusammenschrauben der beiden Spritzen durch Hin-und Herschieben der beiden Kolben gemischt. Dabei bilden sich die Retardpartikel, die in der wässrigen Phase dispergiert vorliegen und injektionsbereit sind.

### Beispiel 2

wie Beispiel 1, nur liegt in der Polymerlösung ein Arzneistoff (z.B. Estradiol, Bupivacain oder Leuprorelin) vor (gelöst oder dispergiert).

### Beispiel 3

wie Beispiel 1, nur wird in die Polymerlösung noch eine wässrige Leuprorelinlösung über eine weitere Spritze vor Herstellung der Retardpartikel dispergiert (w/o-Dispersion).

## Patentansprüche

1. Kit zur Herstellung einer gebrauchsfertigen Einzel-oder Mehrfachdosis einer pharmazeutischen Dispersion von Retardpartikeln, enthaltend mindestens zwei zur Herstellung einer Einzel- oder Mehrfachdosis portionierte Vorprodukte, **dadurch gekennzeichnet, dass** mindestens zwei der Vorprodukte flüssig sind und die Retardpartikel durch Mischen der flüssigen Vorprodukte gebildet werden.

2. Kit nach Anspruch 1, **dadurch gekennzeichnet, dass** die Bildung der Retardpartikel durch Mischen der Vorprodukte erst kurz vor dem Gebrauch geschieht.

3. Kit nach einem der vorangehenden Ansprüche, **dadurch gekennzeichnet, dass** die durch Mischen der Vorprodukte gebildeten Retardpartikel nicht aufbereitet, angereichert oder von flüssiger Phase (z. B. durch Trocknen, Zentrifugieren, Dialysieren oder Filtrieren) abgetrennt werden.

4. Kit nach einem der vorangehenden Ansprüche, **dadurch gekennzeichnet, dass** zur Herstellung der Zubereitung und/oder zur Bildung der Retardpartikel mindestens ein weiteres, festes oder flüssiges Vorprodukt verwendet wird.

5. Kit nach einem der vorangehenden Ansprüche, **dadurch gekennzeichnet, dass** die Vorprodukte im Lagerzustand in separaten Kammern innerhalb einer Vorrichtung primärverpackt vorliegen.

6. Kit nach Anspruch 5, **dadurch gekennzeichnet, dass** die Vorrichtung eine Mehrkammerspritze ist, die zum Mischen der Vorprodukte und zur Applikation der gebrauchsfertigen Zubereitung geeignet ist.

7. Kit nach einem der Ansprüche 1 bis 4, **dadurch gekennzeichnet, dass** die Vorprodukte im Lagerzustand in separaten Kammern primärverpackt vorliegen, die zum Mischen der Vorprodukte miteinander verbindbar sind.

8. Kit nach einem der vorangehenden Ansprüche, **dadurch gekennzeichnet, dass** die Vorprodukte steril vorliegen und das Mischen der Vorprodukte aseptisch geschieht.

9. Kit nach einem der vorangehenden Ansprüche, **dadurch gekennzeichnet, dass** sie ein erstes flüssiges Vorprodukt mit einem Trägermaterial enthält, welches durch Mischen der Vorprodukte zur Bildung der Retärdpartikel verfestigt wird.

10. Kit nach einem der vorangehenden Ansprüche, **dadurch gekennzeichnet, dass** das Trägermaterial ein in wässrigen Phasen lösliches, partiell lösliches oder unlösliches Polymer, ein biokompatibles und/oder bioabbaubares Polymer, ein Protein, ein Lipid oder Wachs, oder eine Mischung mehrerer dieser Substanzen ist.

11. Kit nach Anspruch 10, **dadurch gekennzeichnet, dass** das Trägermaterial Cellulosederivate (z. B. Celluloseacetat, Celluloseacetatphthalat, Ethylcellulose, Hydroxypropylmethylcellulose), Acrylsäurederivate (z. B. Eudragite, Poly(methylmethacrylat), Cyanoacrylate), biokompatible und bioabbaubare Polymere wie Polyanhydride, Polyester (z. B. Polylaktid, Polyglykolid, Polycaprolacton, Polyhydroxybutyrat oder -valerat oder Derivate dieser Polymere, z. B. Poly(laktid-co-glykolid/Polyethylen-glykol Blockpolymere), Polyamine, Polyaminosäuren, Polyorthoester, Polyurethane, Poly(ortho)-carbonate, Polyphosphazene, Polyacetale, Polyketale, Polyoxyethylen-oxypropylene, Polysaccharide (z. B. Na Alginate, Chitosan oder Chitin), Polyethylenglycol, Polyvinylpyrrolidon, und Copolymere oder Terpolymere oder eine Mischung aus zwei oder mehreren der genannten Trägermaterialien umfaßt.

12. Kit nach Anspruch 11, **dadurch gekennzeichnet, dass** das Trägermaterial ein Polylaktid oder ein Polylaktid-Derivat umfasst.

13. Kit nach einem der vorangehenden Ansprüche, **dadurch gekennzeichnet, dass** eines der flüssigen Vorprodukte ein Trägermaterial und ggf. ein Lösemittel oder Lösemittelgemisch enthält, und dass das Trägermaterial und/oder das Lösemittel oder Lösemittelgemisch mit einem zweiten flüssigen Vorprodukt nicht öder nur begrenzt mischbar ist.

14. Kit nach einem der vorangehenden Ansprüche, **dadurch gekennzeichnet, dass** das Trägermaterial in einem Lösemittel oder Lösmittelgemisch vorliegt, welches Wasser und/oder ein organisches'Lösungsmittel wie Ethanol, Aceton, Methylethylketon, Butanol, Ethylformat, Essigsäure, Milchsäure, Pentanol, n- oder i-Propanol, Tetrahydrofuran, Triethylcitrat, Citratester, Phthalatester, Isopropylmyristat, Triacetin, Tributyrin, Propylenglykol, Glycerol, Polyethylenglykol, 2-Ethoxyethylacetat, Ethylacetat, Methylacetat, Ethyllactat, Ethylbutyrat, Benzylalkohol, Benzylbenzoat, Dimethylformamid, Dimethylsulfoxid, Dimethylacetamid, Propylencarbonat, Glykofurol, Solketal, Ölsäure, 2-Pyrrolidon, N-Methyl-2-Pyrrolidon, Caprolactam oder ein Öl oder einen Weichmacher oder eine Mischung aus zwei oder mehreren der genannten Lösungsmittel enthält.

15. Kit nach einem der Ansprüche 9 bis 14, **dadurch gekennzeichnet, dass** sie ein zweites flüssiges Vorprodukt mit einem natürlichen, halbsynthetischen oder synthetischen Fett, Öl oder Wachs, z. B. Baumwollsaatöl, Sojaöl, Saffloröl, hydriertes Erdnußöl, Olivenöl, Rizinusöl, Triglyceridgemische (wie Miglyol), Monoglyceride (wie Glycerolmonooleat), Diglyceride, Silikonöl, Isopropylmynstat, Ethyloleat, Paraffine, Wasser, Glycerol, Propylenglycol, Polyethylenglycol oder eine Mischung aus zwei oder mehreren der genannten Stoffe und ggf. weiteres Trägermaterial enthält.

16. Kit nach Anspruch 15, **dadurch gekennzeichnet, dass** das zweite flüssige Vorprodukt Nasser als einen Hauptbestandteil enthält.

17. Kit nach einem der vorangehenden Ansprüche, **dadurch gekennzeichnet, dass** in mindestens einem der zur Herstellung der Zubereitung und/oder der Retardpartikel verwendeten Vorprodukte ein oder mehrere Wirkstoffe in gelöster, emulgierter oder suspendierter Form enthalten sind.

18. Kit nach einem der vorangehenden Ansprüche, **dadurch gekennzeichnet, dass** der Wirkstoff ein Arzneistoff, ein Peptid- oder Proteinarzneistoff, ein Oligonucleotid oder ein Gentherapeutikum ist.

19. Kit nach Anspruch 18, **dadurch gekennzeichnet, dass** der Arzneistoff aus der Gruppe der Antibiotika, Antiinflammatoria, Antiinfektiva, Antiparasitäre Arzneistoffe, Hormone, immunologisch wirksamen Stoffe, Impfstoffe, Immunmodulatoren, Immunsuppresiva, Zytostatika, Diuretika, Magen-Darm-Mittel, Herz-Kreislauf-Mittel, Respirationstrakt-Mittel, Wachstumsfaktoren, Interferone, Analgetika, Lokalänaesthetika, und/oder Neuropharmaka stammt.

20. Kit nach einem der vorangehenden Ansprüche, **dadurch gekennzeichnet, dass** sie Hilfsstoffe wie z. B. viskositätserhöhende Substanzen, Substanzen zur Erzeugung eines thixotropen Effekts, Stabilisatoren, Emulgatoren, Freigaberegulatoren, Substanzen, welche die Verweildauer am Applikationsort verändern, bioadhäsive Materialien, Penetrationsverbesserer, Substanzen, die die Löslichkeit des Wirkstoffes verringern, oder Substanzen, die die Partikelbildung beeinflussen, enthält.

21. Kit nach einem der vorangehenden Ansprüche, **dadurch gekennzeichnet, dass** sie zur parenteralen, peroralen, rectalen, buccalen, ophthalmischen, pulmonalen, vaginalen, nasalen, lokalen, sublingualen, peridontalen, topischen, intraläsionalen oder transdermalen Anwendung geeignet ist.

22. Kit nach einem der vorangehenden Ansprüche, **dadurch gekennzeichnet, dass** sie zur Behandlung von Weich-und Hartgewebedefekten, Wunden, zur Gewebeerneuerüng, als. Gewebekleber oder zur Füllung von Körperkavitäten geeignet ist.

23. Verfahren zur Herstellung einer gebrauchsfertigen Einzel- oder Mehrfachdosis einer pharmazeutischen Dispersion von Retardpartikeln, **dadurch gekennzeichnet, dass** in einem oder mehreren Schritten die Vorprodukte eines Kits gemäß Ansprüche 1-22 zur Bildung von Retardpartikeln gemischt werden.

24. Verwendung eines Kits gemäß Ansprüche 1-22 zur Herstellung einer Dispersion von Retardpartikeln.

## Claims

1. Kit for the production of a ready-for-use single or multiple dose of a pharmaceutical dispersion of sustained release particles, comprising at least two preliminary products in portions suitable for the production of a single or multiple dose, **characterised in that** at least two of the preliminary products are liquid and the sustained release particles are formed by mixing of the liquid preliminary products.

2. Kit according to claim 1, **characterised in that** the formation of the sustained release particles takes place by mixing of the preliminary products shortly before use.

3. Kit according to any one of the preceding claims, **characterised in that** the sustained release particles formed by mixing of the preliminary products are not processed, concentrated or separated from liquid phase (e.g. by drying, centrifugation, dialysis or filtration).

4. Kit according to any one of the preceding claims, **characterised in that** at least one further solid or liquid preliminary product is used for the production of the preparation and/or for the formation of the sustained release particles.

5. Kit according to any one of the preceding claims, **characterised in that**, in the stored state, the preliminary products are present in separate chambers, as primary packaging, within a device.

6. Kit according to claim 5, **characterised in that** the device is a multi-chamber syringe suitable for the mixing of the preliminary products and for administration of the ready-for-use preparation.

7. Kit according to any one of claims 1 to 4, **characterised in that**, in the stored state, the preliminary products are present in separate chambers, as primary packaging, which can be connected to one another for mixing of the preliminary products.

8. Kit according to any one of the preceding claims, **characterised in that** the preliminary products are in sterile form and mixing of the preliminary products takes place aseptically.

9. Kit according to any one of the preceding claims, **characterised in that** it contains a first liquid preliminary product comprising a carrier material, which is solidified by mixing of the preliminary products to form the sustained release particles.

10. Kit according to any one of the preceding claims, **characterised in that** the carrier material is a polymer that is soluble, partially soluble or insoluble in aqueous phases, a biocompatible and/or biodegradable polymer, a protein, a lipid or wax, or a mixture of a plurality of these substances.

11. Kit according to claim 10, **characterised in that** the carrier material comprises cellulose derivatives (e.g. cellulose acetate, cellulose acetate phthalate, ethylcellulose, hydroxypropylmethylcellulose), acrylic acid derivatives (e.g. Eudragits, poly(methyl methacrylate), cyanoacrylates), biocompatible and biodegradable polymers, such as polyanhydrides, polyesters (e.g. polylactide, polyglycolide, polycaprolactone, polyhydroxy-butyrate or -valerate or derivatives of these polymers, e.g. poly(lactide co-glycolide/polyethylene glycol block polymers), polyamines, polyamino acids, polyorthoesters, polyurethanes, poly(ortho)-carbonates, polyphosphazenes, polyacetals, polyketals, polyoxyethylene-oxypropylenes, polysaccharides (e.g. Na alginates, chitosan or chitin), polyethylene glycol, polyvinylpyrrolidone, and copolymers or terpolymers or a mixture of two or more of the mentioned carrier materials.

12. Kit according to claim 11, **characterised in that** the carrier material comprises a polylactide or a polylactide derivative.

13. Kit according to any one of the preceding claims, **characterised in that** one of the liquid preliminary products contains a carrier material and optionally a solvent or solvent mixture, and **in that** the carrier material and/or the solvent or solvent mixture is not miscible or is miscible to only a limited extent with a second liquid preliminary product.

14. Kit according to any one of the preceding claims, **characterised in that** the carrier material is present in a solvent or solvent mixture comprising water and/or an organic solvent such as ethanol, acetone, methyl ethyl ketone, butanol, ethyl formate, acetic acid, lactic acid, pentanol, n- or iso-propanol, tetrahydrofuran, triethyl citrate, citrate ester, phthalate ester, isopropyl myristate, triacetin, tributyrin, propylene glycol, glycerol, polyethylene glycol, 2-ethoxyethyl acetate, ethyl acetate, methyl acetate, ethyl lactate, ethyl butyrate, benzyl alcohol, benzyl benzoate, dimethylformamide, dimethyl sulfoxide, dimethylacetamide, propylene carbonate, glycofurol, solketal, oleic acid, 2-pyrrolidone, N-methyl-2-pyrrolidone, caprolactam or an oil or a plasticiser or a mixture of two or more of the mentioned solvents.

15. Kit according to any one of claims 9 to 14, **characterised in that** it contains a second liquid preliminary product comprising a natural, semisynthetic or synthetic fat, oil or wax, for example cottonseed oil, soybean oil, safflower oil, hydrogenated groundnut oil, olive oil, castor oil, triglyceride mixtures (such as miglyol), monoglycerides (such as glycerol monooleate), diglycerides, silicone oil, isopropyl myristate, ethyl oleate, paraffins, water, glycerol, propylene glycol, polyethylene glycol or a mixture of two or more of the mentioned substances, and optionally further carrier material.

16. Kit according to claim 15, **characterised in that** the second liquid preliminary product contains water as a main constituent.

17. Kit according to any one of the preceding claims, **characterised in that** in at least one of the preliminary products used for the production of the preparation and/or of the sustained release particles, one or more active ingredients are present in dissolved, emulsified or suspended form.

18. Kit according to any one of the preceding claims, **characterised in that** the active ingredient is a medicament, a peptide or protein medicament, an oligonucleotide or a gene therapy agent.

19. Kit according to claim 18, **characterised in that** the medicament comes from the group of the antibiotics, antiinflammatories, antiinfectives, antiparasitic medicaments, hormones, immunologically active substances, vaccines, immunomodulators, immunosuppressants, cytostatics, diuretics, gastrointestinal agents, cardiovascular agents, respiratory tract agents, growth factors, interferons, analgesics, local anaesthetics, and/or neuropharmaceuticals.

20. Kit according to any one of the preceding claims, **characterised in that** it contains auxiliary substances such as, for example, viscosity-increasing substances, substances for producing a thixotropic effect, stabilisers, emulsifiers, release regulators, substances that change the dwell time at the site of administration, bioadhesive materials, penetration-improving agents, substances that reduce the solubility of the active ingredient, or substances that affect particle formation.

21. Kit according to any one of the preceding claims, **characterised in that** it is suitable for parenteral, peroral, rectal, buccal, ophthalmic, pulmonary, vaginal, nasal, local, sublingual, periodontal, topical, intralesional or transdermal administration.

22. Kit according to any one of the preceding claims, **characterised in that** it is suitable for the treatment of soft- and hard-tissue defects, wounds, for tissue regeneration, as a tissue adhesive or for filling body cavities.

23. Method of producing a ready-for-use single or multiple dose of a pharmaceutical dispersion of sustained release particles, **characterised in that**, in one or more steps, the preliminary products of a kit according to claims 1 to 22 are mixed to form sustained release particles.

24. Use of a kit according to claims 1 to 22 in the production of a dispersion of sustained release particles.

## Revendications

1. Kit de préparation d'une dose unique ou multiple prête à l'emploi d'une dispersion pharmaceutique de particules à libération retardée contenant au moins deux préproduits en des quantités permettant de préparer une dose unique ou multiple, **caractérisé en ce que** deux au moins des préproduits sont liquides et **en ce que** les particules à libération retardée sont formées en mélangeant les préproduits liquides.

2. Kit selon la revendication 1, **caractérisé en ce que** la formation des particules à libération retardée se fait en mélangeant les préproduits juste avant leur utilisation,

3. Kit selon l'une des revendications précédentes, **caractérisé en ce que** les particules à libération retardée qui sont formées en mélangeant les préproduits ne sont pas retraitées, enrichies ou séparées de la phase liquide (par exemple par séchage, centrifugation, dialyse ou filtration).

4. Kit selon l'une des revendications précédentes, **caractérisé en ce qu'**au moins un autre préproduit solide ou liquide est utilisé pour confectionner la préparation et/ou pour former les particules à libération retardée.

5. Kit selon l'une des revendications précédentes, **caractérisé en ce que**, dans l'état stocké, les préproduits sont contenus en guise d'emballage primaire dans des chambres séparées à l'intérieur d'un dispositif.

6. Kit selon la revendication 5, **caractérisé en ce que** le dispositif est une seringue à plusieurs chambres qui est appropriée pour mélanger les préproduits ainsi que pour appliquer la préparation prête à l'emploi.

7. Kit selon l'une des revendications 1 à 4, **caractérisé en ce que**, dans l'état stocké, les préproduits sont contenus en guise d'emballage primaire dans des chambres séparées qui peuvent être assemblées pour mélanger les préproduits.

8. Kit selon l'une des revendications précédentes, **caractérisé en ce que** les préproduits se présentent sous une forme stérile et **en ce que** le mélange des préproduits se fait en conditions aseptiques.

9. Kit selon l'une des revendications précédentes, **caractérisé en ce qu'**il contient un premier préproduit liquide avec un matériau porteur qui se solidifie lorsque les préproduits sont mélangés pour former les particules à libération retardée.

10. Kit selon l'une des revendications précédentes, **caractérisé en ce que** le matériau porteur est un polymère soluble, partiellement soluble ou insoluble dans des phases aqueuses, un polymère biocompatible et/ou biodégradable, une protéine, un lipide ou une cire, ou bien un mélange de plusieurs de ces substances.

11. Kit selon la revendication 10, **caractérisé en ce que** le matériau porteur comprend des dérivés de cellulose (par exemple l'acétate de cellulose, l'acétate phtalate de cellulose, l'éthylcellulose, l'hydroxypropylméthyl cellulose), des dérivés de l'acide acrylique (par exemple des Eudragit, le polyméthacrylate de méthyle, des cyanoacrylates), des polymères biocompatibles et biodégradables tels que des polyanhydrides, des polyesters (par exemple le polylactide, le polyglycolide, la polycaprolactone, le polyhydroxybutyrate ou -valérate ou des dérivés de ces polymères, par exemple des polymères séquencés de poly(lactide-co-glycolide)/polyéthylène glycol, des polyamines, des polyaminoacides, des polyorthoesters, des polyuréthanes, des poly(ortho)carbonates, des polyphosphazènes, des polyacétals, des polycétals, le polyoxyéthylène/oxypropylène, des polysaccharides (par exemple l'alginate de sodium, le chitosane ou la chitine), le polyéthylène glycol, la polyvinylpyrrolidone, ainsi que des copolymères ou terpolymères ou un mélange de deux ou plusieurs des matériaux porteurs précités.

12. Kit selon la revendication 11, **caractérisé en ce que** le matériau porteur comprend un polylactide ou un dérivé de polylactide.

13. Kit selon l'une des revendications précédentes, **caractérisé en ce que** l'un des préproduits liquides contient un matériau porteur et le cas échéant un solvant ou un mélange de solvants et **en ce que** le matériau porteur et/ou le solvant ou le mélange de solvants est non miscible ou seulement miscible de manière restreinte avec un deuxième préproduit liquide.

14. Kit selon l'une des revendications précédentes, **caractérisé en ce que** le matériau porteur est présent dans un solvant ou un mélange de solvants contenant de l'eau et/ou un solvant organique tel que l'éthanol, l'acétone, la méthyléthylcétone, le butanol, le formiate d'éthyle, l'acide acétique, l'acide lactique, le pentanol, le n- ou i-propanol, le tétrahydrofurane, le citrate de triéthyle, un ester citrate, un ester phtalate, le myristate d'isopropyle, la triacétine, la tributyrine, le propylène glycol, le glycérol, le polyéthylène glycol, l'acétate de 2-éthoxyéthyle, l'acétate d'éthyle, l'acétate de méthyle, le lactate d'éthyle, le butyrate d'éthyle, l'alcool benzylique, le benzoate de benzyle, le diméthylformamide, le diméthylsulfoxyde, le diméthylacétamide, le carbonate de propylène, le glycofurol, le solketal, l'acide oléique, la 2-pyrrolidone, la N-méthyl-2-pyrroliclone, le caprolactame ou une huile ou un plastifiant, ou un mélange de deux ou plusieurs des solvants précités.

15. Kit selon l'une des revendications 9 à 14, **caractérisé en ce qu'**il contient un deuxième préproduit liquide avec une matière grasse, une huile ou une cire naturelle, semi-synthétique ou synthétique, par exemple l'huile de graines de coton, l'huile de soja, l'huile de carthame, l'huile d'arachide hydrogénée, l'huile d'olive, l'huile de ricin, des mélanges, de triglycérides (tels que le miglyol), des monoglycérides (tels que le monooléate de glycérol), des diglycérides, l'huile de silicone, le myristate d'isopropyle, l'oléate d'éthyle, des paraffines, l'eau, le glycérol, le propylène glycol, le polyéthylène glycol, ou un mélange de deux ou plusieurs des substances précitées et le cas échéant un matériau porteur.

16. Kit selon la revendication 15, **caractérisé en ce que** le deuxième préproduit liquide contient de l'eau en tant que constituant principal.

17. Kit selon l'une des revendications précédentes, **caractérisé en ce que** l'un au moins des préproduits utilisés pour produire la préparation et/ou les particules à libération retardée contient un ou plusieurs principes actifs sous la forme d'une solution, d'une émulsion ou d'une suspension.

18. Kit selon l'une des revendications précédentes, **caractérisé en ce que** le principe actif est une substance médicamenteuse, une substance médicamenteuse peptidique ou protéinique, un oligonucléotide ou un produit de thérapie génique.

19. Kit selon la revendication 18, **caractérisé en ce que** la substance médicamenteuse est choisie dans le groupe des médicaments antibiotiques, anti-inflammatoires, anti-infectieux, antiparasitaires, des hormones, des substances immunologiquement actives, des vaccins, des immunomodulateurs, des immunosuppresseurs, des cytostatiques, des diurétiques, des agents gastro-intestinaux, des agents cardiaques et circulatoires, des agents destinés au système respiratoire, des facteurs de croissance, des interférons, des analgésiques, des anesthésiques locaux et/ou des agents neuropharmaceutiques.

20. Kit selon l'une des revendications précédentes, **caractérisé en ce qu'**il contient des adjuvants tels que, par exemple, des substances augmentant la viscosité, des substances destinées à produire un effet thixotrope, des agents stabilisants, des agents émulsifiants, des agents régulant la libération, des substances qui modifient le temps de séjour au site d'application, des matériaux bioadhésifs, des renforçateurs de pénétration, des substances qui réduisent la solubilité du principe actif ou des substances qui influent sur la formation des particules.

21. Kit selon l'une des revendications précédentes, **caractérisé en ce qu'**il convient pour une application par voie parentérale, perorale, rectale, buccale, ophtalmique, pulmonaire, vaginale, nasale, locale, sublinguale, parodontale, topique, intralésionnelle ou percutanée.

22. Kit selon l'une des revendications précédentes, **caractérisé en ce qu'**il convient pour le traitement de défauts de tissus mous et durs, de plaies, pour la régénération tissulaire, comme adhésif tissulaire ou pour remplir des cavités de l'organisme.

23. Procédé de préparation d'une dose unique ou multiple prête à l'emploi d'une dispersion pharmaceutique de particules à libération retardée, **caractérisé en ce que** les préproduits d'un kit selon les revendications 1 à 22 sont mélangés en une ou plusieurs étapes afin de former des particules à libération retardée.

24. Utilisation d'un kit selon les revendications 1 à 22 pour préparer une dispersion de particules à libération retardée.
